# EUROPÄISCHE PATENTSCHRIFT

(11) **EP 0 802 810 B1**
(45) Veröffentlichungstag und Bekanntmachung des Hinweises auf die Patenterteilung: **10.10.2001**
(21) Anmeldenummer: 95935793.0
(22) Anmeldetag: 09.11.1995
(51) Int. Cl.: A61M 5/315, A61M 5/24

(54) **Injektionsvorrichtung zur Aufnahme eines flüssigen Medikaments**
Injection device for a liquid medicament
Dispositif d'injection contenant un médicament liquide

(43) Veröffentlichungstag der Anmeldung: 29.10.1997
(73) Patentinhaber: Disetronic Licensing AG, 3400 Burgdorf (CH)
(72) Erfinder: MICHEL, Peter, CH-3400 Burgdorf (CH)
(86) Internationale Anmeldenummer: CH9500261
(87) Internationale Veröffentlichungsnummer: WO9717095

(56) Entgegenhaltungen:
- EP-A- 0 058 536
- EP-A- 0 498 737
- EP-A- 0 594 349

## Beschreibung

Die Erfindung bezieht sich auf eine Injektionsvorrichtung zur Aufnahme eines flüssigen Medikamentes gemäss dem Oberbegriff des Patentanspruchs 1.

Solche Vorrichtungen werden im allgemeinen als "injection-pens" oder kurz "Pens" bezeichnet, wegen ihrer spezifischen Form und Analogie zu einer Schreibfeder.

Im wesentlichen gibt es zwei verschiedene Typen solcher "injection pens" gemäss dem Stand der Technik.

Beispiele für Vorrichtungen gemäss der ersten Gruppe - welche für den Einmalgebrauch bestimmt sind - werden im Detail in den Dokumenten EP-B 327 910, EP-A 594 349 EP-A 496 141 und US 5 295 976 beschrieben. In diesen Vorrichtungen ist die Ampulle, welche das flüssige Medikament enthält, direkt im "injection pen" eingebaut und wird als Einheit an den Patienten geliefert. Folglich werden diese.Vorrichtungen nach deren Erschöpfung als Ganzes weggeworfen, was wegen der komplizierteren Teile des "Pens", welche z.B. auch eine Dosier- und Zählvorrichtung umfassen können, ziemlich kostspielig ist. Vorrichtungen dieser Gruppe sind demzufolge sehr teuer und bieten zudem keine Lösung für die Wiederverwertung von Einzelteilen, wegen der verschiedenen Materialien, welche dazu verwendet werden.

Beispiele für Vorrichtungen der zweiten Gruppe - welche wiederverwendet werden können - sind im Detail in den Dokumenten DE-C 36 38 984, EP-A 498 737 und US 4 883 472 beschrieben. In diesen Vorrichtungen kann die erschöpfte Ampulle durch eine frische ersetzt werden. Allerdings ist bei diesen bekannten Vorrichtungen die Austauschprozedur für die Ampulle ziemlich kompliziert. Um eine neue Ampulle in die Vorrichtung einzusetzen, muss deren Vortriebsmechanismus in eine definierte Ausgangslage zurückgebracht werden (z.B. durch Zurückdrehen der mit einem Gewinde versehenen Kolbenstange). Um diese Prozedur sicher durchführen zu können, muss der Patient entsprechend sorgfältig instruiert werden. Ein weiterer Nachteil besteht in der Gefahr von Fehlfunktionen des Dosiermechanismus, da dieser sehr bald durch den häufigen Gebrauch abgenutzt wird.

Hier will die Erfindung Abhilfe schaffen. Der Erfindung liegt die Aufgabe zugrunde, die obengenannten Nachteile des Standes der Technik zu überwinden.

Die Erfindung löst die gestellte Aufgabe mit einer Injektionsvorrichtung, welche die Merkmale des Anspruchs 1 aufweist.

Der untere, wegwerfbare Teil umfasst diejenigen Elemente, welche sich entweder während des Gebrauchs erschöpfen oder welche weniger kompliziert und deshalb auch weniger kostspielig sind, z.B. als Kunststoff-Teile einen Ampullenhalter zur Aufnahme eines Behälters des flüssigen Medikamentes; einen Nadelhalter, eine Ratsche, eine Gewindemutter und eine mit einem Gewinde versehene Kolbenstange, und als Metallteile eine Feder, welche um die Kolbenstange herum gelagert ist und gegen die Gewindemutter drückt, um diese in ihrer axialen Position zu halten.

Der obere, wiederverwendbare Teil umfasst diejenigen Elemente, welche sich während des Gebrauchs nicht erschöpfen oder welche kompliziert und/oder kostspielig sind, z.B. einen rotierbaren Dosierknopf ein Kupplungselement zur Übertragung der Rotation des Dosierknopfes auf die Gewindemutter ein Gehäuse, welches in lösbarer Weise am unteren Teil befestigbar ist eine (in der Zeichnung nicht dargestellte) Zählvorrichtung, um die Menge des flüssigen Medikamentes zu erfassen und eine mechanische Sicherung.

Der Patient erhält die erfindungsgemässe Vorrichtung in einer zweiteiligen Form, die er vor Gebrauch zusammensetzen kann. Zu diesem Zweck braucht er lediglich den unteren, wegwerfbaren Teil auf den oberen, wiederverwendbaren Teil aufzuklicken oder aufzuschrauben.
Nach Erschöpfung der Ampulle, welche das flüssige Medikament enthält, kann der Patient den gesamten unteren Teil wegwerfen und einen neuen, unteren Teil an den wiederverwendbaren, oberen Teil ankuppeln, welcher die wertvollen Elemente des "injection pen" enthält und während mehrerer Jahre wiederverwendet werden kann. Vorzugsweise besteht der untere Teil vollständig aus rezyklierbaren Materialien, so dass die Umweltbelastung minimal gehalten werden kann.

Ein wichtiger Vorteil der erfindungsgemässen Vorrichtung besteht in der Möglichkeit den oberen Teil zur parallelen Abgabe mehrerer verschiedenartiger Medikamente zu benutzen. Ein weiterer Vorteil besteht in der sicheren Quantifizierung der Medikamentenabgabe. Abhängig vom inneren Durchmesser der Ampulle und der Steigung des Gewindes der Kolbenstange kann die Menge des ausgestossenen Medikamentes exakt durch die Anzahl Umdrehungen definiert werden, wobei z.B. ein Viertel einer vollen Umdrehung einer Nocke der Sicherungs-Vorrichtung entsprechen kann, welche ein Rückwärtsdrehen des Dosier-Knopfes verhindert. Die Maximaldosis pro Injektion wird durch den maximalen Hub des Dosier-Knopfes bestimmt. Deshalb wird die Medikamenten-Dosis für eine bestimmte Therapie durch den unteren, wegwerfbaren Teil der erfindungsgemässen Vorrichtung vorausbestimmt. Folglich ist der obere Teil mit der Dosier-Einheit zu einem einfachen Einstell- und Ausstoss-Mechanismus reduziert, welche bei Bedarf durch einen Zählmechanismus ergänzt werden kann, welcher Rotationsbewegungen (z.B. Viertelsdrehungen) erfassen kann.

Weitere vorteilhafte Ausgestaltungen der Erfindung sind in den abhängigen Ansprüchen gekennzeichnet.

Die Erfindung und Weiterbildungen der Erfindung werden im folgenden anhand der teilweise schematischen Darstellungen eines Ausführungsbeispieles noch näher erläutert.

Es zeigen:
Fig. 1 einen Längsschnitt durch eine erfindungsgemässe Vorrichtung;
Fig. 2 einen Längsschnitt durch den unteren Teil einer Vorrichtung nach Fig. 1; und
Fig. 3 einen Längsschnitt durch den oberen Teil einer Vorrichtung nach Fig. 1.

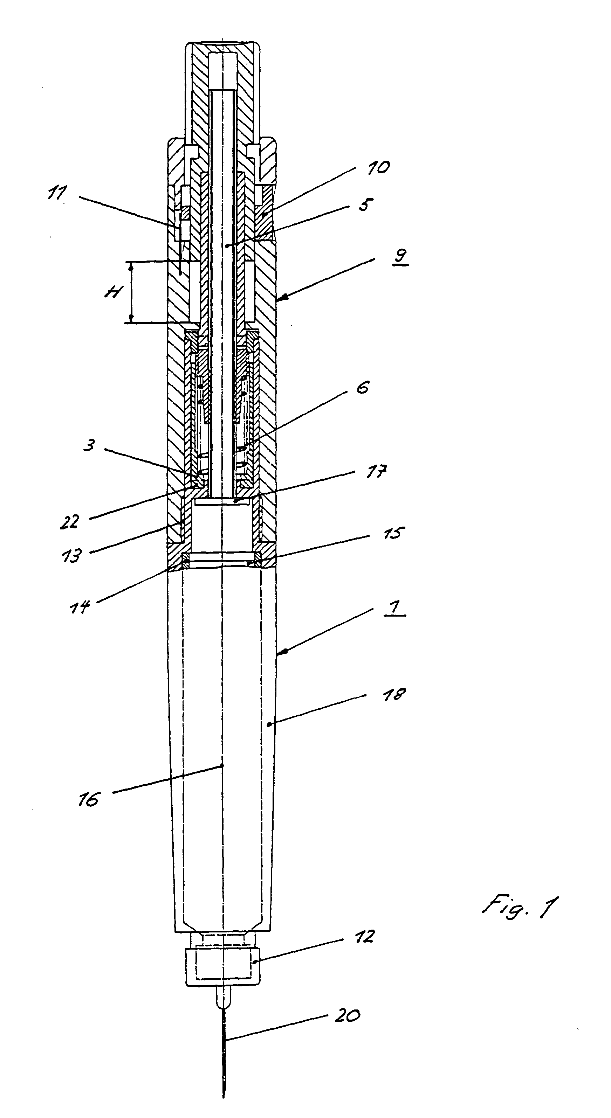

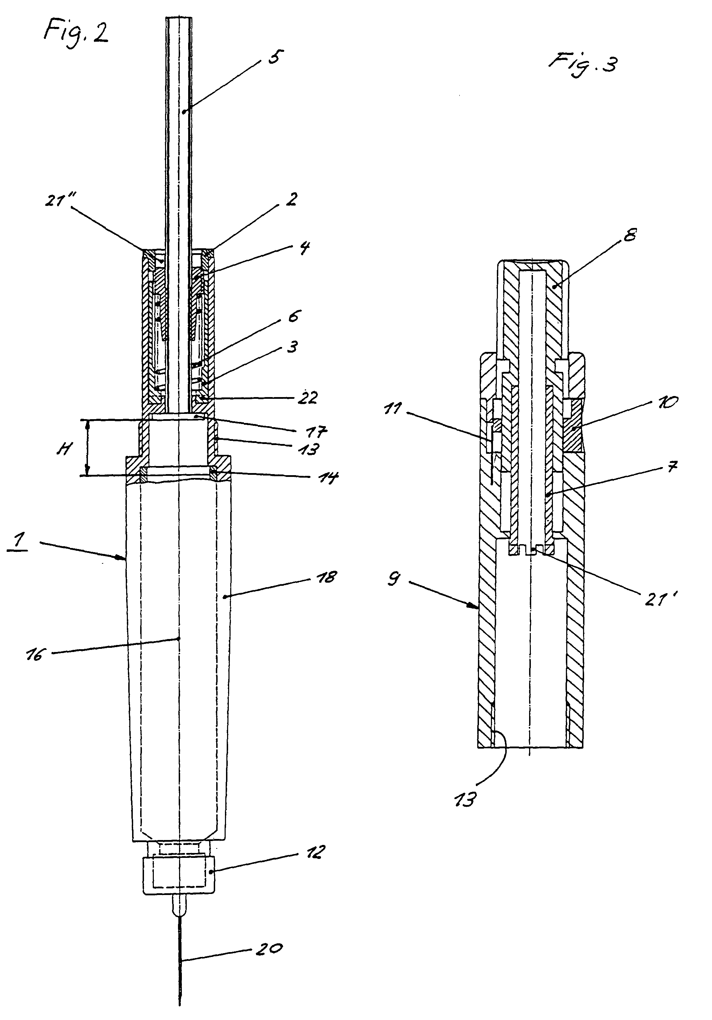

Die in Fig. 1 dargestellte erfindungsgemässe Injektionsvorrichtung besteht aus einem unteren Teil 1 und einem oberen Teil 9 welche mittels einer Gewindeverbindung 13 oder eines Bajonettverschlusses miteinander gekuppelt sind. Der untere Teil 1 ist in Fig. 2 und der obere Teil in Fig. 3 im jeweils entkoppelten Zustand dargestellt.

Wie aus den Fig. 1 und 2 hervorgeht, umfasst der zylindrische Ampullenhalter 18 des unteren Teils 1 den Behälter 14 für das flüssige Medikament. Am vorderen Ende des Ampullenhalters 18 ist der Nadelhalter 12 mit der Nadel 20 aufgeschraubt. Der Behälter 14, welcher aus einer Ampulle bestehen kann, ist an seinem hinteren Ende durch einen axial verschieblichen Stopfen 15 verschlossen. Eine nicht-rotierbare, aber axial verschiebbare Kolbenstange 5 ist in der Längsachse 16 der Injektionsvorrichtung fluchtend angeordnet und kann mittels ihrer Frontplatte 17 auf den Stopfen 15 wirken. Die Kolbenstange 5 hat einen unrunden Querschnitt damit sie nicht-rotierbar ist. Die Gewindemutter 4 umgibt die Kolbenstange 5, wobei die beiden Elemente 4,5 als Spindel/Mutter-Vorrichtung wirken. Zu diesem Zweck hat die Gewindemutter 4 ein Innengewinde, welches dem Gewinde der Kolbenstange 5 entspricht.

Die Gewindemutter 4 ist mit einem sie umgebenden Rohr 3 verbunden, in welchem eine Feder 6 untergebracht ist, welche die Gewindemutter 4 in ihrer axialen Position hält. Die Feder 6 ist an ihrem unteren Ende durch das Rohr 3 gesichert, dessen Durchmesser dort entsprechend verringert ist. Diese Konstruktion garantiert, dass sich bei einer Rotation der Gewindemutter 4 die Feder 6 ebenfalls mitdreht, da die Gewindemutter 4 drehfest aber axial verschiebbar mit dem Rohr 3 verbunden ist.
Die Gewindemutter 4 ist an ihrem oberen Ende durch einen Flansch 2 gesichert. Am unteren Ende des Rohres 3 ist eine Ratsche 22 angebracht, welche weiter unten noch im Detail beschrieben wird.

Ein Hohlzylinder 7, der einstückig mit einem rotierbaren und über eine vorbestimmte Hublänge längsverschiebbaren Dosierknopf 8 des oberen Teils 9 verbunden ist, weist an seinem freien Ende eine Kupplung 21' auf; entsprechen ist die Gewindemutter 4 im unteren Teil 1 an ihrem hinteren Ende mit einer damit zusammenwirkenden Kupplung 21" versehen. Wenn der obere Teil 9 und der untere Teil 1 kombiniert werden, so greifen die Kupplungen 21' und 21'' ineinander.

Nachfolgend wird die Funktionsweise des Injektionssystems beschrieben, ausgehend von der in Fig. 1 dargestellten Ruheposition. Der Benutzer dreht den Knopf 8 und überträgt damit die Drehung auf den Hohlzylinder 7, welcher mittels der Kupplungen 21' und 21'' drehbar an die Gewindemutter 4 gekoppelt ist. Da die Gewindemutter 4 durch die Feder 6 in ihrer axialen Stellung gehalten wird, erfolgt eine axiale Vorwärtsbewegung der Kolbenstange 5 durch die Drehung der Gewindemutter 4. Die Anzahl Drehungen N (z.B. Viertelsdrehungen) des Knopfes 8 wird durch einen geeigneten-zeichnerisch nicht dargestellten - Zählmechanismus erfasst, welcher entweder mechanisch oder elektronisch arbeitet. Gleichzeitig gibt die als Ratsche 22 ausgebildete Rückdrehsicherung (am unteren Ende des Rohres 3) ein akustisches oder taktiles Signal, z.B. nach jeder Viertelsumdrehung des Knopfes 8.

Gemäss der Anzahl N der Umdrehungen wird die Frontplatte 17 um den entsprechenden Betrag Nz näher zum Stopfen 15 hin bewegt, wobei z der vorausbestimmbaren Anzahl diskreter Schritte entspricht.

Nach der oben beschriebenen Einstellung der Injektionsdosis wird der Knopf 8 in seiner gesamten vorausbestimmten Hublänge H hinuntergedrückt. Wenn die Kolbenstange 5 um den Betrag Nz nach vorwärts bewegt worden ist, wird sich die Frontplatte 17 vorerst um den Längenbetrag (H-Nz) nach vorwärts bewegen, bis sie den Stopfen 15 berührt und sich danach um den verbleibenden Betrag Nz des Hubs weiterbewegen, unter Ausschüttung des Volumens V = N×z×F des flüssigen Medikamentes, wobei F der inneren Querschnittsfläche des Behälters 14 entspricht.

Falls der Knopf 8 überhaupt nicht gedreht wird, erfolgt kein Ausstoss an Medikamentenflüssigkeit, weil die Frontplatte 17 den Stopfen 15 erst nach Zurücklegung der vollständigen Hublänge H berührt.

Falls Knopf .8 in seiner gesamten Hublänge H nach unten gedrückt wird, wird er durch eine Sicherung 10 mittels der Blattfeder 11 in seiner Endposition festgehalten, wie dies aus Fig. 3 ersichtlich ist.

Bevor der Patient den Kopf 8 drehen kann (um eine neue Medikamentendosis einzustellen), muss er die Sicherung 10 an der Seite des Injektions-Pens drücken, so dass die Feder 6 die Frontplatte 17 zurückdrücken kann, wobei die Kolbenstange 5, die Gewindemutter 4, der Hohlzylinder 7 und der Knopf 8 in ihre Ausgangspositionen zurückkehren. Erst jetzt ist das Injektionssystem für eine neue Einstellung einer Medikamenten-dosis bereit.

## Patentansprüche

1. Injektionsvorrichtung zur Aufnahme eines flüssigen Medikamentes, welches mittels einer Nadel (20) in den menschlichen oder tierischen Körper injizierbar ist, umfassend:
- einen Ampullenhalter (18) zur Aufnahme eines, das flüssige Medikament enthaltenden, Behälters (14), welcher einen axial beweglichen Stopfen (15) vom Querschnitt F aufweist, zur Ausstossung des flüssigen Medikamentes aus dem Behälter (14);
- eine kinematische Vorrichtung zum axialen Vortrieb des Stopfens (15) über eine Anzahl N von Einzelschritten z, wobei die Anzahl N eine natürliche Zahl bedeutet;
- ein lösbar mit dem Ampullenhalter (18) verbindbares Gehäuse mit einem rotierbaren und über eine vorbestimmte Hublänge längsverschieblichen Dosierknopf (8) zur Auswahl der Anzahl N von Einzelschritten z und mit einstückig mit dem Dosierknopf (8) verbundenen Mitteln (7) zur Betätigung der kinematischen Vorrichtung, wodurch eine wählbare Ausstossung eines Volumens V = N x z x F des flüssigen Medikamentes aus dem Behälter (14) bewirkbar ist, **dadurch gekennzeichnet, dass** der Ampullenhalter (18) einen unteren, wegwerfbaren Teil (1) bildet, die kinematische Vorrichtung im unteren Teil (1) angeordnet ist , und das Gehäuse einen oberen, wiederverwendbaren Teil (9) bildet.

2. Injektionsvorrichtung nach Anspruch 1, **dadurch gekennzeichnet, dass** die kinematische Vorrichtung nur in einer Richtung bewegbar ist und zwar vom oberen Teil (9) zum unteren Teil (1).

3. Injektionsvorrichtung nach Anspruch 1 oder 2, **dadurch gekennzeichnet, dass** die kinematische Vorrichtung des unteren Teils (1) zusätzlich die folgenden Teile umfasst:
- eine rotationsfeste, aber axial bewegliche Kolbenstange (5), welche auf den Stopfen (15) des Behälters (14) wirkt;
- ein auf der Kolbenstange (5) angebrachtes Aussengewinde (23);
- eine Gewindemutter (4) mit einem Innengewinde, welches mit dem Aussengewinde (23) zusammenwirkt.

4. Injektionsvorrichtung nach einem der Ansprüche 1 - 3, **dadurch gekennzeichnet, dass** sowohl der untere Teil (1) als auch der obere Teil (9) mit Kupplungsmitteln zur gegenseitigen lösbaren Fixierung versehen sind.

5. Injektionsvorrichtung nach einem der Ansprüche 1 - 4,
**dadurch gekennzeichnet, dass** die mit dem rotierbaren Dosierknopf (8) verbundenen Mittel (7)
ein Kupplungselement (21') zur Übertragung der Rotation des Dosierknopfes (8) zur kinematischen Vorrichtung des unteren Teils (1) aufweisen.

6. Injektionsvorrichtung nach einem der Ansprüche 3 - 5, **dadurch gekennzeichnet, dass** der untere Teil (1) zusätzlich eine Feder (6) umfasst, welche um die Kolbenstange (5) herum angeordnet ist und durch ihre Kompression die Gewindemutter (4) in ihrer axialen Position festhält.

7. Injektionsvorrichtung nach einem der Ansprüche 1 - 6, **dadurch gekennzeichnet, dass** am Dosierknopf (8) oder den damit verbundenen Mittel (7) zusätzlich ein elektronischer oder mechanischer Zähler zur Auswahl und Überwachung der Anzahl N von Einzelschritten z angeordnet ist.

8. Injektionsvorrichtung nach einem der Ansprüche 1 - 7, **dadurch gekennzeichnet, dass** der untere Teil (1) vollständig aus rezyklierbarem Material besteht.

## Claims

1. An injection device for accommodating a liquid drug which can be injected into the human or animal body by means of a needle (20), including:
- an ampoule holder (18) for accommodating a container (14) which contains the liquid drug and which has an axially movable plunger (15) of cross-section F, for expelling the liquid drug from the container (14);
- a kinematic device for axially advancing the plunger (15) over a number N of individual steps z, wherein the number N denotes a natural number; and
- a housing which can be releasably connected to the ampoule holder (18) and having a metering knob (8) which is rotatable and longitudinally slidable over a predetermined stroke length for selection of the number N of individual steps z and having means (7) integrally connected to the metering knob (8) for actuation of the kinematic device, whereby selective expulsion of a volume V = N x z x F of the liquid drug from the container (14) can be effected,
**characterised in that** the ampoule holder (18) forms a lower disposable portion (1), the kinematic device is arranged in the lower portion (1), and the housing forms an upper re-usable portion (9).

2. An injection device according to claim 1 **characterised in that** the kinematic device is movable only in one direction, more specifically from the upper portion (9) to the lower portion (1).

3. An injection device according to claim 1 or claim 2 **characterised in that** the kinematic device of the lower portion (1) additionally includes the following parts:
- a rotationally fixed but axially movable plunger rod (5) which acts on the plunger (15) of the container (14);
- a male screwthread (23) provided on the plunger rod (5); and
- a screwthreaded nut (4) having a female screwthread co-operating with the male screwthread (23).

4. An injection device according to one of claims 1 to 3 **characterised in that** both the lower portion (1) and also the upper portion (9) are provided with coupling means for mutual releasable fixing.

5. An injection device according to one of claims 1 to 4 **characterised in that** the means (7) connected to the rotatable metering knob (8) have a coupling element (21') for transmitting the rotary movement of the metering knob (8) to the kinematic device of the lower portion (1).

6. An injection device according to one of claims 3 to 5 **characterised in that** the lower portion (1) additionally includes a spring (6) which is arranged around the plunger rod (5) and which by compression thereof holds the screwthreaded nut (4) fast in its axial position.

7. An injection device according to one of claims 1 to 6 **characterised in that** additionally arranged on the metering knob (8) or the means (7) connected thereto is an electronic or mechanical counter for selecting and monitoring the number N of individual steps z.

8. An injection device according to one of claims 1 to 7 **characterised in that** the lower portion (1) completely consists of recyclable material.

## Revendications

1. Dispositif d'injection pour la prise d'un médicament liquide, susceptible d'être injecté au moyen d'une aiguille (20) dans le corps humain ou animal, comprenant :
- un porte-ampoule (18) pour recevoir un récipient (14) qui contient le médicament liquide et comporte un bouchon axialement mobile (15) de section F, afin d'éjecter le médicament liquide hors du récipient (14) ;
- un dispositif cinématique pour faire avancer axialement le bouchon (15) sur un certain nombre N de pas individuels z, le nombre N étant un nombre naturel ;
- un boîtier relié de manière détachable au porte-ampoule (18), comportant un bouton de dosage (8) rotatif et capable de translation longitudinale sur une longueur prédéterminée, pour la sélection du nombre N de pas individuels z, et comportant des moyens (7) reliés d'une seule pièce avec le bouton de dosage (8) pour l'actionnement du dispositif cinématique, grâce à quoi on peut réaliser une éjection sélectionnée d'un volume V = N . z . F du médicament liquide hors du récipient (14),
**caractérisé en ce que** le porte-ampoule (18) forme une partie inférieure à jeter (1), **en ce que** le dispositif cinématique est agencé dans la partie inférieure (1), et **en ce que** le boîtier forme une partie supérieure (9) réutilisable.

2. Dispositif d'injection selon la revendication 1, **caractérisé en ce que** le dispositif cinématique n'est déplaçable que dans une seule direction, et cela depuis la partie supérieure (9) vers la partie inférieure (1).

3. Dispositif d'injection selon la revendication 1 ou 2, **caractérisé en ce que** le dispositif cinématique de la partie inférieure (1) comprend additionnellement les parties suivantes :
- une tige de piston (5) solidaire en rotation mais mobile en sens axial, qui agit sur le bouchon (15) du récipient (14) ;
- un filetage extérieur (23) ménagée sur la tige de piston (5) ; et
- un écrou taraudé (4) avec un taraudage qui coopère avec le filetage (23).

4. Dispositif d'injection selon l'une quelconque des revendications 1 à 3, **caractérisé en ce que** la partie inférieure (1) tout comme la partie supérieure (9) est pourvue de moyens d'accouplement pour leur fixation mutuelle détachable.

5. Dispositif d'injection selon l'une quelconque des revendications 1 à 4, **caractérisé en ce que** les moyens (7) reliés au bouton de dosage rotatif (8) comprennent un élément d'accouplement (21') pour la transmission de la rotation du bouton de dosage (8) au dispositif cinématique de la partie inférieure (1).

6. Dispositif d'injection selon l'une quelconque des revendications 3 à 5, **caractérisé en ce que** la partie inférieure (1) comprend en supplément un ressort (6), celui-ci étant agencé autour de la tige de piston (5) et retenant par sa compression l'écrou taraudé (4) dans sa position axiale.

7. Dispositif d'injection selon l'une quelconque des revendications 1 à 6, **caractérisé en ce qu'**il est prévu en supplément un compteur électronique ou mécanique, agencé sur le bouton de dosage (8) ou sur les moyens (7) reliés à celui-ci, pour la sélection et la surveillance du nombre N de pas individuels z.

8. Dispositif d'injection selon l'une quelconque des revendications 1 à 7, **caractérisé en ce que** la partie inférieure (1) est entièrement réalisée en matériau recyclable.
